# EUROPEAN PATENT APPLICATION

(11) **EP 2 573 082 A1**
(43) Date of publication of application: **27.03.2013**
(21) Application number: 12192439.3
(22) Date of filing: 30.12.2008
(51) Int. Cl.: C07D 401/12, A61K 31/4439, A61P 1/00

(54) **CRYSTALLINE SOLVATED FORMS OF (R)-2-[[[3-METHYL-4-(2,2,2-TRIFLUOROETHOXY)-2-PYRIDINYL]METHYL]SULFINYL]-1H-BENZIMIDAZOLE**

(30) Priority: 31.12.2007 US 18021 P
(62) Divisional of application: 10183275.6
(71) Applicant: Takeda Pharmaceutical Company Limited, Osaka 541-0045 (JP)
(72) Inventor: Urakami, Koji, Osaka, 532-0024 (JP); Lorimer, Keith, Indiana, IN Indiana 47906 (US); Meyer, Keith, Indiana, IN Indiana 47906 (US); Andres, Mark, Christopher, Indiana, IN Indiana 47906-1927 (US)
(74) Representative: Kingsbury, Oliver William

(57) **Abstract**

The novel methanol solvate, ethanol solvate, ethanol·hydrate and isopropanol·hydrate crystals of (R)-2-[[[3-methyl-4-(2,2,2-trifluoroethoxy)-2-pyridinyl]methyl]sulfinyl]-1H-benzimidazole; pharmaceutical agents which comprise these crystals; and their use for the prophylaxis or treatment of digestive ulcer.

## Description

### TECHNICAL FIELD OF THE INVENTION

The present invention relates to a crystal of a benzimidazole compound showing an antiulcer action.

### BACKGROUND OF THE INVENTION

2-[[[3-Methyl-4-(2,2,2-trifluoroethoxy)-2-pyridinyl]methyl]sulfinyl]-1H-benzimidazole or a salt thereof having an antiulcer action has been reported in JP-A-61-50978, etc.

An anhydrous or hydrate crystal of optically active (R)-2-[[[3-methyl-4-(2,2,2-trifluoroethoxy)-2-pyridinyl]methyl]sulfinyl]-1H-benzimidazole has been reported in JP-A-2001-058990, JP-A-2002-037783, JP-A-2002-226478 and the like.

### SUMMARY OF THE *INVENTION

The present inventors have conducted intensive studies of a novel crystal of (R)-2-[[[3-methyl-4-(2,2,2-trifluoroethoxy)-2-pyridinyl]methyl]sulfinyl]-1H-benzimidazole currently sold all over the world as a pharmaceutical product having a superior antiulcer activity, and found a novel hydrate crystal, a novel methanol solvate crystal, a novel ethanol solvate crystal, a novel ethanol·hydrate crystal, and a novel isopropanol·hydrate crystal, and also found that these crystals unexpectedly show different physical properties (solubility, transfer stability), particularly properties of solubility, although they contain the same drug ingredient as the conventional crystals of optically active forms. Since the solubility of a drug may influence the bioavailability due to the pharmaceutical agent side during the gastrointestinal absorption process, the crystals of the present invention can be designed differently as a preparation from the conventional crystals. Moreover, these crystals can be synthetic intermediates for crystals of a pharmaceutical product having superior antiulcer activity, (*R*)-2-[[[3-methyl-4-(2,2,2-trifluoroethoxy)-2-pyridinyl]methyl]sulfinyl]-1H-benzimidazole. They have found that these crystals serve satisfactorily as pharmaceuticals or synthetic intermediates for pharmaceuticals. Based on these findings, they have completed the present invention.

Accordingly, the present invention relates to:
[1] a hydrate crystal of (*R*)-2-[[[3-methyl-4-(2,2,2-trifluoroethoxy)-2-pyridinyl]methyl]sulfinyl]-1H-benzimidazole wherein the X-ray powder diffraction analysis pattern has characteristic peaks at interplanar spacings (d) of 9.62±0.2, 8.90±0.2, 5.93±0.2, 5.66±0.2 and 5.04±0.2 Angstrom; (Form II crystal)
[2] an ethanol·hydrate crystal of (*R*)-2-[[[3-methyl-4-(2,2,2-trifluoroethoxy)-2-pyridinyl]methyl]sulfinyl]-1H-benzimidazole wherein the X-ray powder diffraction analysis pattern has characteristic peaks at interplanar spacings (d) of 13.23±0.2, 6.21±0.2, 4.75±0.2, 4.51±0.2 and 4.41±0.2 Angstrom; (Form III crystal)
[3] an isopropanol·hydrate crystal of (*R*)-2-[[[3-methyl-4-(2,2,2-trifluoroethoxy)-2-pyridinyl]methyl]sulfinyl]-1H-benzimidazole wherein the X-ray powder diffraction analysis pattern has characteristic peaks at interplanar spacings (d) of 14.90±0.2, 5.01±0.2, 4.56±0.2, 4.26±0.2 and 3.50±0.2 Angstrom; (Form IV crystal)
[4] a hydrate crystal of (R)-2-[[[3-methyl-4-(2,2,2-trifluoroethoxy)-2-pyridinyl]methyl]sulfinyl]-1H-benzimidazole wherein the X-ray powder diffraction analysis pattern has characteristic peaks at interplanar spacings (d) of 9.21±0.2, 6.70±0.2, 5.88±0.2, 4.83±0.2 and 4.40±0.2 Angstrom; (Pattern V crystal)
[5] a hydrate crystal of (R)-2-[[[3-methyl-4-(2,2,2-trifluoroethoxy)-2-pyridinyl]methyl]sulfinyl]-1H-benzimidazole wherein the X-ray powder diffraction analysis pattern has characteristic peaks at interplanar spacings (d) of 8.86±0.2, 8.43±0.2, 5.60±0.2, 5.22±0.2 and 4.83±0.2 Angstrom; (Form VI crystal)
[6] a methanol solvate crystal of (R)-2-[[[3-methyl-4-(2,2,2-trifluoroethoxy)-2-pyridinyl]methyl]sulfinyl]-1H-benzimidazole wherein the X-ray powder diffraction analysis pattern has characteristic peaks at interplanar spacings (d) of 13.42±0.2, 13.22±0.2, 6.21±0.2, 6.16±0.2, 4.51±0.2 and 4.32±0.2 Angstrom;
[7] an ethanol solvate crystal of (R)-2-[[[3-methyl-4-(2,2,2-trifluoroethoxy)-2-pyridinyl]methyl]sulfinyl]-1H-benzimidazole wherein the X-ray powder diffraction analysis pattern has characteristic peaks at interplanar spacings (d) of 13.71+0.2, 13.50+0.2, 13.22+0.2, 13.06±0.2 and 6.16±0.2 Angstrom;
[8] a 1.0 hydrate crystal of (*R*)-2-[[[3-methyl-4-(2,2,2-trifluoroethoxy)-2-pyridinyl]methyl]sulfinyl]-1H-benzimidazole wherein the X-ray powder diffraction analysis pattern has characteristic peaks at interplanar spacings (d) of 8.93±0.2, 8.47±0.2, 5.65+0.2, 5.63±0.2 and 5.25±0.2 Angstrom;
[9] a 1.5 hydrate crystal of (*R*)-2-[[[3-methyl-4-(2,2,2-trifluoroethoxy)-2-pyridinyl]methyl]sulfinyl]-1H-benzimidazole wherein the X-ray powder diffraction analysis pattern has characteristic peaks at interplanar spacings (d) of 5.95±0.2, 5.91±0.2, 5.65+0.2, 4.51±0.2 and 4.50±0.2 Angstrom;
[10] a pharmaceutical agent which comprises the crystal of any of the above-mentioned [1] to [9];
[11] a pharmaceutical agent according to the above [10], which is an agent for the prophylaxis or treatment of digestive ulcer, and so forth.

### BRIEF DESCRIPTION OF THE DRAWINGS

Figure 1 shows X-ray powder diffraction patterns of solvate crystals of (*R*)-2-[[[3-methyl-4-(2,2,2-trifluoroethoxy)-2-pyridinyl]methyl]sulfinyl]-1H-benzimidazole.
Figure 2 shows FT-Raman spectrums of solvate crystals of (*R*)-2-[[[3-methyl-4-(2,2,2-trifluoroethoxy)-2-pyridinyl]methyl]sulfinyl]-1H-benzimidazole.
Figure 3 shows solid ¹³C-NMR spectrums of solvate crystals of (*R*)-2-[[[3-methyl-4-(2,2,2-trifluoroethoxy)-2-pyridinyl]methyl]sulfinyl]-1H-benzimidazole.
Figure 4 shows X-ray powder diffraction patterns of methanol solvate crystal and ethanol solvate crystal of (R)-2-[[[3-methyl-4-(2,2,2-trifluoroethoxy)-2-pyridinyl]methyl]sulfinyl]-1H-benzimidazole.
Figure 5 shows X-ray powder diffraction patterns of hydrate crystals of (R)-2-[[[3-methyl-4-(2,2,2-trifluoroethoxy)-2-pyridinyl]methyl]sulfinyl]-1H-benzimidazole.
Figure 6 is a chart showing concentration vs. time for Forms I, II, III, IV and VI of R(+)-lansoprazole in water under constant agitation at up to 25°C.
Figure 7 is a scheme showing the relationships among Forms I, II, III, IV and VI of R(+)-lansoprazole.

### DETAILED DESCRIPTION OF THE INVENTION

A hydrate crystal of (R)-2-[[[3-methyl-4-(2,2,2-trifluoroethoxy)-2-pyridinyl]methyl]sulfinyl]-1H-benzimidazole includes 0.5 hydrate to 5.0 hydrate. Among others, 0.5 hydrate, 1.0 hydrate, 1.5 hydrate, 2.0 hydrate and 2.5 hydrate are preferred. More preferred is 0.5 hydrate, 1.0 hydrate or 1.5 hydrate. In addition, a hydrate of (*R*)-2-[[[3-methyl-4-(2,2,2-trifluoroethoxy)-2-pyridinyl]methyl]sulfinyl]-1H-benzimidazole may be deuterium substituted.

As an alcohol solvate crystal of (R)-2-[[[3-methyl-4-(2,2,2-trifluoroethoxy)-2-pyridinyl]methyl]sulfinyl]-1H-benzimidazole, for example, methanol solvate crystal, ethanol solvate crystal, propanol solvate crystal, isopropanol solvate crystal and the like can be mentioned, and methanol solvate crystal, ethanol solvate crystal, isopropanol solvate crystal and the like are preferable, and methanol solvate crystal and ethanol solvate crystal are particularly preferable.

An alcohol solvate crystal of (R)-2-[[[3-methyl-4-(2,2,2-trifluoroethoxy)-2-pyridinyl]methyl]sulfinyl]-1H-benzimidazole includes 0.1 alcohol solvate to 3.0 alcohol solvate.

Specific examples of the methanol solvate crystal and ethanol solvate crystal include 0.4 to 0.6 methanol solvate, 0.5 to 0.7 ethanol solvate and the like, and 0.5 methanol solvate and 0.6 ethanol solvate are particularly preferable.

A solvate crystal of (R)-2-[[[3-methyl-4-(2,2,2-trifluoroethoxy)-2-pyridinyl]methyl]sulfinyl]-1H-benzimidazole may be formed using two or more kinds of solvents, and an embodiment wherein the crystal is formed using two kinds of solvents is preferable.

When a solvate crystal is formed using two or more kinds of solvents, the solvents are selected from alcohol (methanol, ethanol, propanol, isopropanol and the like), water and the like. Preferably, a solvate crystal is formed using alcohol and water, more preferably ethanol and water, or isopropanol and water. In the present invention, for example, "a solvate crystal formed using ethanol and water" is indicated as an "ethanol·hydrate crystal".

When a solvate crystal is formed using two or more kinds of solvents, the molar ratio of the total amount of solvents used relative to (R)-2-[[[3-methyl-4-(2,2,2-trifluoroethoxy)-2-pyridinyl]methyl]sulfinyl]-1H-benzimidazole is generally selected from the range of 0.1 mol to 3.0 mol.

When a solvate crystal is formed using two or more kinds of solvents, while the constitution ratio of the solvent is not particularly limited, it is selected from the range of alcohol:water = 1:0.5 to 1:3.0, in the case of, for example, an alcohol·hydrate crystal.

As a solvate crystal formed using two or more kinds of solvents, an ethanol·hydrate crystal or an isopropanol·hydrate crystal is preferable. Specific examples include a 0.5 to 0.9 ethanol·0.8 to 1.2 hydrate crystal and a 0.5 to 0.9 isopropanol·1.0 to 1.4 hydrate crystal, with particular preference given to a 0.7 ethanol·1 hydrate crystal and a 0.7 isopropanol·1.2 hydrate crystal.

The hydrate crystal, methanol solvate crystal, ethanol solvate crystal, ethanol·hydrate crystal and isopropanol· hydrate crystal of (R)-2-[[[3-methyl-4-(2,2,2-trifluoroethoxy)-2-pyridinyl]methyl]sulfinyl]-1H-benzimidazole of the present invention can be produced by subjecting 2-[[[3-methyl-4-(2,2,2-trifluoroethoxy)-2-pyridinyl]methyl]sulfinyl]-1H-benzimidazole or a salt thereof to an optical resolution or subjecting 2-[[[3-methyl-4-(2,2,2-trifluoroethoxy)-2-pyridinyl]methyl]thio]-1H-benzimidazole to an asymmetrical oxidization to obtain the (R)-isomer, followed by crystallizing the resultant isomer, or transforming the known crystal of the (R)-isomer.

Methods of optical resolution include per se known methods, for example, a fractional recrystallization method, a chiral column method, a diastereomer method, and so forth. Asymmetric oxidation includes per se known method.

The "fractional recrystallization method" includes a method in which a salt is formed between a racemate and an optically active compound [e.g., (+) -mandelic acid, (-)-mandelic acid, (+) -tartaric acid, (-)-tartaric acid, (+)-1-phenethylamine, (-)-1-phenethylamine, cinchonine, (-)-cinchonidine, brucine, etc.], which salt is separated by fractional recrystallization etc., and, if desired, subjected to a neutralization process, to give a free optical isomer.

The "chiral column method" includes a method in which a racemate or a salt thereof is applied to a column for optical isomer separation (chiral column). In the case of liquid chromatography, for example, optical isomers are separated by adding a racemate to a chiral column such as ENANTIO-OVM (produced by Tosoh Corporation) or the DAICEL CHIRAL series (produced by Daicel Corporation), and developing the racemate in water, a buffer (e.g., phosphate buffer), an organic solvent (e.g., hexane, ethanol, methanol, isopropanol, acetonitrile, trifluoroacetic acid, diethylamine, triethylamine, etc.), or a solvent mixture thereof. In the case of gas chromatography, for example, a chiral column such as CP-Chirasil-DeX CB (produced by GL Science) is used to separate optical isomers.

The "diastereomer method" includes a method in which a racemate and an optically active reagent are reacted (preferably, an optically active reagent is reacted to the 1-position of the benzimidazole group) to give a diastereomer mixture, which is then subjected to ordinary separation means (e.g., fractional recrystallization, chromatography, etc.) to obtain either diastereomer, which is subjected to a chemical reaction (e.g., acid hydrolysis, base hydrolysis, hydrogenolysis, etc.) to cut off the optically active reagent moiety, whereby the desired optical isomer is obtained. Said "optically active reagent" includes, for example, an optically active organic acids such as MTPA [α-methoxy-α-(trifluoromethyl)phenylacetic acid] and (-)-menthoxyacetic acid; and an optically active alkoxymethyl halides such as (1R-endo)-2-(chloromethoxy)-1,3,3-trimethylbicyclo[2.2.1]heptane, etc.

2-[[[3-Methyl-4-(2,2,2-trifluoroethoxy)-2-pyridinyl]methyl]sulfinyl]-1H-benzimidazole or a salt thereof is produced by the methods described in JP-A-61-50978, USP 4,628,098 etc. or analogous methods thereto.

Methods of crystallization include per se known methods, for example, a crystallization from solution.

Methods of the "crystallization from solution" include, for example, a concentration method, a slow cooling method, a reaction method (diffusion method, electrolysis method), a hydrothermal growth method, a fusing agent method, and so forth. Solvents to be used include, for example, aromatic hydrocarbons (e.g., benzene, toluene, xylene, etc.), halogenated hydrocarbons (e.g., dichloromethane, chloroform, etc.), saturated hydrocarbons (e.g., hexane, heptane, cyclohexane, etc.), ethers (e.g., diethyl ether, diisopropyl ether, tetrahydrofuran, dioxane, etc.), nitriles (e.g., acetonitrile, etc.), ketones (e.g., acetone, etc.), sulfoxides (e.g., dimethylsulfoxide, etc.), acid amides (e.g., N,N-dimethylformamide, etc.), esters (e.g., ethyl acetate, etc.), alcohols (e.g., methanol, ethanol, isopropyl alcohol, etc.), water, and so forth. When a hydrate crystal is to be obtained, water, a mixture of water and other solvent, and the like are used; when an alcohol solvate crystal is to be obtained, alcohol or a mixture of alcohol and other solvent is used; and when an alcohol·hydrate crystal is to be obtained, a mixture of alcohol and water or a mixture of alcohol, water and other solvent is used.

When a mixed solvent of two or more kinds is to be used, they are mixed at a suitable ratio (e.g., 1:1 to 1:100) and used. Preferably, two or more kinds of solvents are mixed at a ratio of 1:1 to 1:20, more preferably the ratio of water:other solvent is 1:1, 1:9 or 9:1 (e.g., the ratio of water:methanol is 1:1, the ratio of water:ethanol is 1:9, the ratio of water:acetone is 9:1, the ratio of water:ethanol is 9:1).

Known crystals to be used for transformation from known crystals include the anhydrous crystal and hydrate crystal described in JP-A-2001-058990, hydrate crystal described in JP-A-2002-037783, anhydrous crystal described in JP-A-2002-226478 and the like.

(*R*)-2-[[[3-methyl-4-(2,2,2-trifluoroethoxy)-2-pyridinyl]methyl]sulfinyl]-1H-benzimidazole 1.5 hydrate crystal (after-mentioned Form II) can be produced by a production method characterized by a process of agitating a mixture of (*R*)-2-[[[3-methyl-4-(2,2,2-trifluoroethoxy)-2-pyridinyl]methyl]sulfinyl]-1H-benzimidazole (preferably, anhydrous crystal of (*R*)-2-[[[3-methyl-4-(2,2,2-trifluoroethoxy)-2-pyridinyl]methyl]sulfinyl]-1H-benzimidazole), and water and other solvent (e.g., acetone, ethanol etc.) at a mixing ratio of 1:1 to 100:1 (preferably, 1:1 to 20:1, more preferably, 9:1) at an ambient temperature for 2 to 4 days (preferably, 3days ± 6 to 12 hrs, more preferably, 3 days) by constant rotation. As the "other solvent" in the mixture, acetone is preferable.

As other production method, (*R*)-2-[[[3-methyl-4-(2,2,2-trifluoroethoxy)-2-pyridinyl]methyl]sulfinyl]-1H-benzimidazole 1.5 hydrate crystal (1.5 hydrate crystal of the after-mentioned (2)) can be produced by a production method characterized by a process of crystallization from a mixture of (*R*)-2-[[[3-methyl-4-(2,2,2-trifluoroethoxy)-2-pyridinyl]methyl]sulfinyl]-1H-benzimidazole (preferably, anhydrous crystal of (*R*)-2-[[[3-methyl-4-(2,2,2-trifluoroethoxy)-2-pyridinyl]methyl]sulfinyl]-1H-benzimidazole), and water and other solvent (e.g., acetone, methanol etc.) at a mixing ratio of 1:1 to 1:20 (preferably, 1:1) by standing the mixture at -25 to -15 °C (preferably, -20°C ± 1°C, more preferably, -20 °C ± 0.5 °C, still more preferably, -20 °C). The crystals obtained by the production method are dried under reduced pressure for 2 day to 4 days (preferably, 3 days ± 6 to 12 hrs, more preferably, 3 days) to give (*R*)-2-[[[3-methyl-4-(2,2,2-trifluoroethoxy)-2-pyridinyl]methyl]sulfinyl]-1H--benzimidazole 1.0 hydrate crystal (the after-mentioned 1.0 hydrate crystal). As the "other solvent" in the mixture, methanol is preferable.

(R)-2-[[[3-Methyl-4-(2,2,2-trifluoroethoxy)-2-pyridinyl]methyl]sulfinyl]-1H-benzimidazole 0.5 hydrate crystal (the after-mentioned Form VI)can be produced by a production method characterized by drying (*R*)-2-[[[3-methyl-4-(2,2,2-trifluoroethoxy)-2-pyridinyl]methyl]sulfinyl]-1H-benzimidazole 1.5 hydrate crystal (the above-mentioned Form II) at an ambient temperature under vacuum. As the "drying" under vacuum, drying for 24 hrs ± 6 to 12 hrs (preferably, overnight) is preferable.

(*R*)-2-[[[3-Methyl-4-(2,2,2-trifluoroethoxy)-2-pyridinyl]methyl]sulfinyl]-1H-benzimidazole methanol solvate crystal (the after-mentioned methanol solvate crystal) can be produced by a production method characterized by a process of crystallization from a solution obtained by adding methanol to (*R*)-2-[[[3-methyl-4-(2,2,2-trifluoroethoxy)-2-pyridinyl]methyl]sulfinyl]-1H-benzimidazole at room temperature (preferably, anhydrous crystal of (R)-2-[[[3-methyl-4-(2,2,2-trifluoroethoxy)-2-pyridinyl]methyl]sulfinyl]-1H-benzimidazole).

(*R*)-2-[[[3-Methyl-4-(2,2,2-trifluoroethoxy)-2-pyridinyl]methyl]sulfinyl]-1H-benzimidazole ethanol solvate crystal (the after-mentioned ethanol solvate crystal) can be produced by a production method characterized by a process of crystallization from a solution obtained by adding ethanol to (R)-2-[[[3-methyl-4-(2,2,2-trifluoroethoxy)-2-pyridinyl]methyl]sulfinyl]-1H-benzimidazole (preferably, anhydrous crystal of (R)-2-[[[3-methyl-4-(2,2,2-trifluoroethoxy)-2-pyridinyl]methyl]sulfinyl]-1H-benzimidazole) at room temperature.

(R)-2-[[[3-Methyl-4-(2,2,2-trifluoroethoxy)-2-pyridinyl]methyl]sulfinyl]-1H-benzimidazole ethanol hydrate crystal (preferably, about 0.7 ethanol·1 hydrate) (the after-mentioned Form III) can be produced by a production method characterized by a process of dissolving (*R*)-2-[[[3-methyl-4-(2,2,2-trifluoroethoxy)-2-pyridinyl]methyl]sulfinyl]-1H-benzimidazole (preferably, anhydrous crystal of (R)-2-[[[3-methyl-4-(2,2,2-trifluoroethoxy)-2-pyridinyl]methyl]sulfinyl]-1H-benzimidazole) in a mixture of water and ethanol at a dissolution ratio of 1:1 to 1:20 (preferably, 1:9), and precipitation from the solution.

(R)-2-[[[3-Methyl-4-(2,2,2-trifluoroethoxy)-2-pyridinyl]methyl]sulfinyl]-1H-benzimidazole isopropanol hydrate crystal (preferably, about 0.7 isopropanol·1.2 hydrate) (after-mentioned Form IV) can be produced by a production method characterized by a process of filtering a solution of (R)-2-[[[3-methyl-4-(2,2,2-trifluoroethoxy)-2-pyridinyl]methyl]sulfinyl]-1H-benzimidazole (preferably, anhydrous crystal of (R)-2-[[[3-methyl-4-(2,2,2-trifluoroethoxy)-2-pyridinyl]methyl]sulfinyl]-1H-benzimidazole) in isopropanol and evaporating the filtrate under ambient conditions to allow crystallization. As the "filtering", filtering with a 0.1 to 0.5 µm (preferably, 0.2 µm) filter is preferable.

(*R*)-2-[[[3-methyl-4-(2,2,2-trifluoroethoxy)-2-pyridinyl]methyl]sulfinyl]-1H-benzimidazole hydrate crystal (the after-mentioned Form V) can be produced by a production method characterized by a process of agitating a mixture of (*R*)-2-[[[3-methyl-4-(2,2,2-trifluoroethoxy)-2-pyridinyl]methyl]sulfinyl]-1H-benzimidazole (preferably, anhydrous crystal of (*R*)-2-[[[3-methyl-4-(2,2,2-trifluoroethoxy)-2-pyridinyl]methyl]sulfinyl]-1H-benzimidazole), and water and other solvent (e.g., acetone, ethanol etc.) at a mixing ratio of 1:1 to 100:1 (preferably, 1:1 to 20:1, more preferably, 9:1) at an ambient temperature for 2 to 4 days (preferably, 3days ± 6 to 12 hrs, more preferably, 3 days) by constant rotation. As the "other solvent" in the mixture, ethanol is preferable.

As a method of crystal transformation, crystallization from the above-mentioned solution, as well as, for example, a transpiration method (known crystal is dissolved in a solvent and, after filtration, the solvent is evaporated under ambient conditions), a slurry method (known crystal is added to a solvent such that excess solid remains to give a suspension, the suspension is stirred at ambient temperature or under heating and the solid is collected by filtration), drying under reduced pressure, trituration, pressurization and the like can be mentioned.

For analyzing the crystal obtained, X-ray diffraction crystallographic analysis is commonly used. In addition, crystal orientation can also be determined by a mechanical method, an optical method (e.g., FT-Raman spectrum, solid NMR spectrum), etc.

The peak of the spectrum obtained by the above-mentioned analysis method inevitably contains a certain measurement error by its nature. A crystal with a spectrum peak within the error range is also encompassed in the crystal of the present invention. For example, "±0.2" in the interplanar spacing (d) of powder X-ray diffraction means that the error is tolerable.

As (*R*)-2-[[[3-methyl-4-(2,2,2-trifluoroethoxy)-2-pyridinyl]methyl]sulfinyl]-1H-benzimidazole solvate crystal, a 1.5 hydrate crystal wherein the X-ray powder diffraction analysis pattern has characteristic peaks at interplanar spacings (d) of 9.62±0.2, 8.90±0.2, 5.93±0.2, 5.66±0.2 and 5.04±0.2 Angstrom, preferably, a 1.5 hydrate crystal wherein the X-ray powder diffraction analysis pattern has characteristic peaks at interplanar spacings (d) of 9.62±0.2, 8.90±0.2, 8.07±0.2, 6.63±0.2, 6.01±0.2, 3.93±0.2, 5.66±0.2, 5.04±0.2, 4.50±0.2 and 3.00±0.2 Angstrom, more preferably, a 1.5 hydrate crystal wherein the X-ray powder diffraction analysis pattern has characteristic peaks at interplanar spacings (d) of 9.62±0.2, B.90±0.2, 8.07±0.2, 6.63±0.2, 6.01±0.2, 5.93±0.2, 5.66±0.2, 5.04±0.2, 4.50±0.2, 3.51±0.2 and 3.00±0.2 Angstrom (hereinafter referred to as Form II crystal), an about 0.7 ethanol·1 hydrate crystal wherein the X-ray powder diffraction analysis pattern has characteristic peaks at interplanar spacings (d) of 13.23±0.2, 6.21±0.2, 4.75±0.2, 4.51±0.2 and 4.41±0.2 Angstrom, preferably, an about 0.7 ethanol·1 hydrate crystal wherein the X-ray powder diffraction analysis pattern has characteristic peaks at interplanar spacings (d) of 13.23±0.2, 6.21±0.2, 5.06±0.2, 4.97±0.2, 4.75±0.2, 4.51±0.2, 4.41±0.2 and 4.32±0.2 Angstrom (hereinafter referred to as Form III crystal), an about 0.7 isopropanol·1.2 hydrate crystal wherein the X-ray powder diffraction analysis pattern has characteristic peaks at interplanar spacings (d) of 14.90±0.2, 5.01±0.2, 4.56±0.2, 4.26±0.2 and 3.50±0.2 Angstrom, preferably an about 0.7 isopropanol·1.2 hydrate crystal wherein the X-ray powder diffraction analysis pattern has characteristic peaks at interplanar spacings (d) of 14.90±0.2, 6.66±0.2, 5.01±0.2, 4.56+0.2, 4.50±0.2, 4.36±0.2, 4.26±0.2, 3.90±0.2, 3.63±0.2 and 3.50±0.2 Angstrom (hereinafter referred to as Form IV crystal), a hydrate crystal wherein the X-ray powder diffraction analysis pattern has characteristic peaks at interplanar spacings (d) of 9.21±0.2, 6.70±0.2, 5.88±0.2, 4.83±0.2 and 4.40±0.2 Angstrom, preferably a hydrate crystal wherein the X-ray powder diffraction analysis pattern has characteristic peaks at interplanar spacings (d) of 9.21±0.2, 8.30±0.2, 6.70±0.2, 6.12±0.2, 5.88+0.2, 4.83±0.2, 4.71±0.2, 4.66±0.2, 4.40±0.2 and 3.18±0.2 Angstrom, more preferably, a hydrate crystal wherein the X-ray powder diffraction analysis pattern has characteristic peaks at interplanar spacings (d) of 20.03±0.2, 13.25±0.2, 9.50±0.2, 9.21±0.2, 8.30±0.2, 6.80±0.2, 6.70±0.2, 6.12±0.2, 5.88±0.2, 5.71±0.2, 5.62±0.2, 4.88±0.2, 4.83±0.2, 4.71±0.2, 4.66±0.2, 4.50±0.2, 4.40±0.2, 4.15±0.2, 4.13±0.2, 4.09±0.2, 3.93±0.2, 3.69±0.2, 3.66±0.2, 3.62±0.2, 3.47±0.2, 3.42±0.2, 3.39±0.2, 3.21±0.2, 3.18±0.2, 3.14±0.2, 3.12±0.2, 3.10±0.2, 2.77±0.2, 2.67±0.2, 2.43±0.2 and 2.42±0.2 Angstrom (hereinafter referred to as Pattern V crystal), a 0.5 hydrate crystal wherein the X-ray powder diffraction analysis pattern has characteristic peaks at interplanar spacings (d) of 8.86±0.2, 8.43±0.2, 5.60+0.2, 5.22±0.2 and 4.83±0.2 Angstrom, preferably, a 0.5 hydrate crystal wherein the X-ray powder diffraction analysis pattern has characteristic peaks at interplanar spacings (d) of 8.86±0.2, 8.43±0.2, 5.60±0.2, 5.22±0.2, 4.83±0.2 and 4.21±0.2 Angstrom (hereinafter referred to as Form VI crystal) and the like can be mentioned, with preference given to Form II crystal, Form III crystal, Form IV crystal, Pattern V crystal and Form VI crystal.

As (*R*)-2-[[[3-methyl-4-(2,2,2-trifluoroethoxy)-2-pyridinyl]methyl]sulfinyl]-1H-benzimidazole methanol solvate crystal, a methanol solvate crystal wherein the X-ray powder diffraction analysis pattern has characteristic peaks at interplanar spacings (d) of 13.42±0.2, 13.22±0.2, 6.21±0.2, 6.16±0.2, 4.51±0.2 and 4.32±0.2 Angstrom, preferably, a methanol solvate crystal wherein the X-ray powder diffraction analysis pattern has characteristic peaks at interplanar spacings (d) of 13.76±0.2, 13.42±0.2, 13.22±0.2, 6.21±0.2, 6.16±0.2, 4.97±0.2, 4.87±0.2, 4.74±0.2, 4.51±0.2, 4.32±0.2 and 3.98±0.2 Angstrom, more preferably, a methanol solvate crystal wherein the X-ray powder diffraction analysis pattern has characteristic peaks at interplanar spacings (d) of 14.24±0.2, 14.06±0.2, 13.76±0.2, 13.42±0.2, 13.22±0.2, 10.13±0.2, 7.32±0.2, 6.24±0.2, 6.21±0.2, 6.16±0.2, 5.63±0.2, 5.13±0.2, 5.06±0.2, 4.97±0.2, 4.89±0.2, 4.87±0.2, 4.74±0.2, 4.53±0.2, 4.51±0.2, 4.41±0.2, 4.32±0.2, 4.13±0.2, 4.10±0.2, 4.08±0.2, 3.99±0.2, 3.98±0.2, 3.73±0.2, 3.64±0.2, 3.43±0.2, 3.41±0.2, 3.35(3.3533)±0.2 and 3.35(3.3483)±0.2 Angstrom can be mentioned.

As (R)-2-[[[3-methyl-4-(2,2,2-trifluoroethoxy)-2-pyridinyl]methyl]sulfinyl]-1H-benzimidazole ethanol solvate crystal, an ethanol solvate crystal wherein the X-ray powder diffraction analysis pattern has characteristic peaks at interplanar spacings (d) of 13.71±0.2, 13.50±0.2, 13.22±0.2, 13.06±0.2 and 6.16±0.2 Angstrom, preferably, an ethanol solvate crystal wherein the X-ray powder diffraction analysis pattern has characteristic peaks at interplanar spacings (d) of 13.89±0.2, 13.71±0.2, 13.50±0.2, 13.22±0.2, 13.06±0.2, 6.22±0.2, 6.16±0.2, 4.74±0.2, 4.32±0.2 and 4.31±0.2 Angstrom, more preferably, an ethanol solvate crystal wherein the X-ray powder diffraction analysis pattern has characteristic peaks at interplanar spacings (d) of 14.29±0.2, 13.89±0.2, 13.71±0.2, 13.50±0.2, 13.22±0.2, 13.06±0.2, 10.09±0.2, 7.32±0.2, 6.22±0.2, 6.16±0.2, 5.14±0.2, 5.09±0.2, 4.98±0.2, 4.97±0.2, 4.88±0.2, 4.84±0.2, 4.78±0.2, 4.74±0.2, 4.65±0.2, 4.62+0.2, 4.58±0.2, 4.53±0.2, 4.52±0.2, 4.51±0.2, 4.49±0.2, 4.44±0.2, 4.39±0.2, 4.35±0.2, 4.33±0.2, 4.32±0.2, 4.31±0.2, 4.09±0.2, 4.07±0.2, 3.97±0.2, 3.95±0.2, 3.75±0.2, 3.74±0.2, 3.63±0.2, 3.44±0.2, 3.43±0.2, 3.42±0.2, 3.38±0.2, 3.36±0.2, 3.35(3.3508)±0.2, 3.35(3.3459)±0.2, 3.34±0.2 and 3.03±0.2 Angstrom can be mentioned.

As (*R*)-2-[[[3-methyl-4-(2,2,2-trifluoroethoxy)-2-pyridinyl]methyl]sulfinyl]-1H-benzimidazole hydrate crystal,
(1) a 1.0 hydrate crystal wherein the X-ray powder diffraction analysis pattern has characteristic peaks at interplanar spacings (d) of 8.93±0.2, 8.47±0.2, 5.65±0.2, 5.63±0.2 and 5.25±0.2 Angstrom,
   preferably, a 1.0 hydrate crystal wherein the X-ray powder diffraction analysis pattern has characteristic peaks at interplanar spacings (d) of 8.93±0.2, 8.47±0.2, 5.65±0.2, 5.63±0.2, 5.60±0.2, 5.25±0.2, 4.86±0.2, 4.85±0.2, 4.23±0.2, 4.11±0.2 and 4.10±0.2 Angstrom,
   more preferably, a 1.0 hydrate crystal wherein the X-ray powder diffraction analysis pattern has characteristic peaks at interplanar spacings (d) of 9.77±0.2, 9.71±0.2, 8.93±0.2, 8,47±0.2, 5.65±0.2, 5.63±0.2, 5.60±0.2, 5.25±0.2, 4.86±0.2, 4.85±0.2, 4.83±0.2, 4.81±0.2, 4.45±0.2, 4.31±0.2, 4.25±0.2, 4.23±0.2, 4.15±0.2, 4.14±0.2, 4.11±0.2, 4.10±0.2, 4.08±0.2, 4.07±0.2, 3.98±0.2, 3.95±0.2, 3.68±0.2, 3.65±0.2, 3.53±0.2, 3.38±0.2, 3.36±0.2, 3.23±0.2, 3.16±0.2, 3.09±0.2 and 3.08±0.2 Angstrom and
(2) a 1.5 hydrate crystal wherein the X-ray powder diffraction analysis pattern has characteristic peaks at interplanar spacings (d) of 5.95±0.2, 5.91±0.2, 5.65±0.2, 4.51±0.2 and 4.50±0.2 Angstrom,
   preferably, a 1.5 hydrate crystal wherein the X-ray powder diffraction analysis pattern has characteristic peaks at interplanar spacings (d) of 8.87±0.2, 8.04±0.2, 6.00±0.2, 5.97±0.2, 5.95±0.2, 5.91±0.2, 5.65±0.2, 5.02±0.2, 4.51±0.2 and 4.50±0.2 Angstrom,
   more preferably, a 1.5 hydrate crystal wherein the X-ray powder diffraction analysis pattern has characteristic peaks at interplanar spacings (d) of 13.18±0.2, 9.60±0.2, 9.07±0.2, 9.02±0.2, 8.87±0.2, 8.04±0.2, 6.59±0.2, 6.00±0.2, 5.97±0.2, 5.95±0.2, 5.91±0.2, 5.72±0.2, 5.65±0.2, 5.47±0.2, 5.43±0.2, 5.05±0.2, 5.02±0.2, 5.00±0.2, 4.51±0.2, 4.50±0.2, 4.47±0.2, 4.46±0.2, 4.26±0.2, 4.18±0.2, 4.13±0.2, 4.11±0.2, 3.99±0.2, 3.98±0.2, 3.75±0.2, 3.73±0.2, 3.72±0.2, 3.71±0.2, 3.66±0.2, 3.65±0.2, 3.64±0.2, 3.57±0.2, 3.51(3.5119)±0.2, 3.51(3.5064)±0.2, 3.49±0.2, 3.46±0.2, 3.40±0.2, 3.28±0.2, 3.28±0.2, 3.16±0.2, 3.08±0.2, 3.00±0.2, 2.99±0.2 and 2.88±0.2 Angstrom can be mentioned.

When the same numerical value is recited twice in a row in the above-mentioned d values, it means that two peaks are present at close positions such that the same numerical value is obtained when rounded to two decimal places.

Thus obtained hydrate crystal, methanol solvate crystal, ethanol solvate crystal, ethanol·hydrate crystal and isopropanol·hydrate crystal of (R)-2-[[[3-methyl-4-(2,2,2-trifluoroethoxy)-2-pyridinyl]methyl]sulfinyl]-1H-benzimidazole or a salt thereof (hereinafter also referred to as "crystal of the present invention") are useful as a pharmaceutical because they show excellent antiulcer action, gastric acid secretion-inhibiting action, mucosa-protecting action, anti-*Helicobacter* pylori action, etc., and because they are of low toxicity. Since the crystal of the present invention shows different physical properties (e.g., solubility and the like) from those of conventional (*R*)-2-[[[3-methyl-4-(2,2,2-trifluoroethoxy)-2-pyridinyl]methyl]sulfinyl]-l1H-benzimidazole crystal, a preparation design applying such properties is available. Since the crystal of the present invention has low solubility, preparation, such as a controlled release preparation and the like with sustainability, may be considered. In addition, since the crystal can be a synthetic intermediate for (R)-2-[[[3-methyl-4-(2,2,2-trifluoroethoxy)-2-pyridinyl]methyl]sulfinyl]-1H-benzimidazole crystal, it is useful as a synthetic intermediate for pharmaceutical agents.

The crystal of the present invention is useful in mammals (e.g., humans, monkeys, sheep, bovines, horses, dogs, cats, rabbits, rats, mice, etc.) for the treatment and prevention of peptic ulcer (e.g., gastric ulcer, gastric ulcer due to postoperative stress, duodenal ulcer, anastomotic ulcer, ulcer caused by non-steroidal antiinflammatory agents etc.); Zollinger-Ellison syndrome; gastritis; erosive esophagitis; reflux esophagitis such as erosive reflux esophagitis and the like; symptomatic gastroesophageal reflux disease (symptomatic GERD) such as non-erosive reflux disease or gastroesophageal reflux disease free of esophagitis and the like; functional dyspepsia; gastric cancer (including gastric cancer associated with promoted production of interleukin-1β due to gene polymorphism of interleukin-1); stomach MALT lymphoma; gastric hyperacidity; upper gastrointestinal hemorrhage due to peptic ulcer, acute stress ulcer, hemorrhagic gastritis or invasive stress (e.g. stress caused by major surgery requiring postoperative intensive management, and cerebrovascular disorder, head trauma, multiple organ failure and extensive burn, each requiring intensive treatment) and the like; preanesthetic administration, eradication of *Helicobacter pylori* or eradication assistance and the like.

As used herein, the above-mentioned reflux esophagitis and symptomatic gastroesophageal reflux disease (symptomatic GERD) are sometimes collectively referred to simply as GERD.

The crystal of the present invention is of low toxicity and can be safely administered orally or non-orally (e.g., topical, rectal and intravenous administration, etc.), as such or in the form of pharmaceutical compositions formulated with a pharmacologically acceptable carrier, e.g., tablets (including sugar-coated tablets and film-coated tablets), powders, granules, capsules (including soft capsules), orally disintegrating tablets, orally disintegrating films, liquids, injectable preparations, suppositories, sustained-release preparations and patches, in accordance with a commonly known method.

The content of the crystal of the present invention in the pharmaceutical composition of the present invention is about 0.01 to 100% by weight relative to the entire composition. Varying depending on subject of administration, route of administration, target disease etc., its dose is normally about 0.5 to 1,500 mg/day, preferably about 5 to 150 mg/day, based on the active ingredient, for example, when it is orally administered as an antiulcer agent to an adult human (60 kg). The crystal of the present invention may be administered once daily or in 2 to 3 divided portions per day.

Pharmacologically acceptable carriers that may be used to produce the pharmaceutical composition of the present invention include various organic or inorganic carrier substances in common use as pharmaceutical materials, including excipients, lubricants, binders, disintegrants, water-soluble polymers and basic inorganic salts for solid preparations; and solvents, dissolution aids, suspending agents, isotonizing agents, buffers and soothing agents for liquid preparations. Other ordinary pharmaceutical additives such as preservatives, antioxidants, coloring agents, sweetening agents, souring agents, bubbling agents and flavorings may also be used as necessary.

Such "excipients" include, for example, lactose, sucrose, D-mannitol, starch, cornstarch, crystalline cellulose, light silicic anhydride and titanium oxide.

Such "lubricants" include, for example, magnesium stearate, sucrose fatty acid esters, polyethylene glycol, talc and stearic acid.

Such "binders" include, for example, hydroxypropyl cellulose, hydroxypropylmethyl cellulose, crystalline cellulose, α-starch, polyvinylpyrrolidone, gum arabic powder, gelatin, pullulan and low-substitutional hydroxypropyl cellulose.

Such "disintegrants" include (1) crosslinked povidone, (2) what is called super-disintegrants such as crosslinked carmellose sodium (FMC-Asahi Chemical) and carmellose calcium (Gotoku Yakuhin), (3) carboxymethyl starch sodium (e.g., product of Matsutani Chemical), (4) low-substituted hydroxypropyl cellulose (e.g., product of Shin-Etsu Chemical), (5) cornstarch, and so forth. Said "crosslinked povidone" may be any crosslinked polymer having the chemical name 1-ethenyl-2-pyrrolidinone homopolymer, including polyvinylpyrrolidone (PVPP) and 1-vinyl-2-pyrrolidinone homopolymer, and is exemplified by Colidon CL (produced by BASF), Polyplasdon XL (produced by ISP), Polyplasdon XL-10 (produced by ISP) and Polyplasdon INF-10 (produced by ISP).

Such "water-soluble polymers" include, for example, ethanol-soluble water-soluble polymers [e.g., cellulose derivatives such as hydroxypropyl cellulose (hereinafter also referred to as HPC), polyvinylpyrrolidone] and ethanol-insoluble water-soluble polymers [e.g., cellulose derivatives such as hydroxypropylmethyl cellulose (hereinafter also referred to as HPMC), methyl cellulose and carboxymethyl cellulose sodium, sodium polyacrylate, polyvinyl alcohol, sodium alginate, guar gum].

Such "basic inorganic salts" include, for example, basic inorganic salts of sodium, potassium, magnesium and/or calcium. Preferred are basic inorganic salts of magnesium and/or calcium. More preferred are basic inorganic salts of magnesium. Such basic inorganic salts of sodium include, for example, sodium carbonate, sodium hydrogen carbonate, disodium hydrogenphosphate, etc.

Such basic inorganic salts of potassium include, for example, potassium carbonate, potassium hydrogen carbonate, etc. Such basic inorganic salts of magnesium include, for example, heavy magnesium carbonate, magnesium carbonate, magnesium oxide, magnesium hydroxide, magnesium metasilicate aluminate, magnesium silicate, magnesium aluminate, synthetic hydrotalcite [Mg₆Al₂(OH)₁₆·CO₃·4H₂O], alumina hydroxide magnesium, and so forth. Among others, preferred is heavy magnesium carbonate, magnesium carbonate, magnesium oxide, magnesium hydroxide, etc. Such basic inorganic salts of calcium include, for example, precipitated calcium carbonate, calcium hydroxide, etc.

Such "solvents' include, for example, water for injection, alcohol, propylene glycol, macrogol, sesame oil, corn oil and olive oil.

Such "dissolution aids" include, for example, polyethylene glycol, propylene glycol, D-mannitol, benzyl benzoate, ethanol, trisaminomethane, cholesterol, triethanolamine, sodium carbonate and sodium citrate.

Such "suspending agents" include, for example, surfactants such as stearyltriethanolamine, sodium lauryl sulfate, laurylaminopropionic acid, lecithin, benzalkonium chloride, benzethonium chloride and monostearic glycerol; and hydrophilic polymers such as polyvinyl alcohol, polyvinylpyrrolidone, carboxymethyl cellulose sodium, methyl cellulose, hydroxymethyl cellulose, hydroxyethyl cellulose and hydroxypropyl cellulose.

Such "isotonizing agents" include, for example, glucose, D-sorbitol, sodium chloride, glycerol and D-mannitol.

Such "buffers" include, for example, buffer solutions of phosphates, acetates, carbonates, citrates etc.

Such "soothing agents" include, for example, benzyl alcohol.

Such "preservatives" include, for example, p-oxybenzoic acid esters, chlorobutanol, benzyl alcohol, phenethyl alcohol, dehydroacetic acid and sorbic acid.

Such "antioxidants" include, for example, sulfites, ascorbic acid and α-tocopherol.

Such "coloring agents" include, for example, food colors such as Food Color Yellow No. 5, Food Color Red No. 2 and Food Color Blue No. 2; and food lake colors and red oxide.

Such "sweetening agents" include, for example, saccharin sodium, dipotassium glycyrrhetinate, aspartame, stevia and thaumatin.

Such "souring agents" include, for example, citric acid (citric anhydride), tartaric acid and malic acid.

Such "bubbling agents" include, for example, sodium bicarbonate.

Such "flavorings" may be synthetic substances or naturally occurring substances, and include, for example, lemon, lime, orange, menthol and strawberry.

The crystal of the present invention may be prepared as a preparation for oral administration in accordance with a commonly known method, by, for example, compression-shaping it in the presence of an excipient, a disintegrant, a binder, a lubricant, or the like, and subsequently coating it as necessary by a commonly known method for the purpose of taste masking, enteric dissolution or sustained release. For an enteric preparation, an intermediate layer may be provided by a commonly known method between the enteric layer and the drug-containing layer for the purpose of separation of the two layers.

For preparing the crystal of the present invention as an orally disintegrating tablet, available methods include, for example, a method in which a core containing crystalline cellulose and lactose is coated with the crystal of the present invention and a basic inorganic salt, and is further coated with a coating layer containing a water-soluble polymer, to give a composition, which is coated with an enteric coating layer containing polyethylene glycol, further coated with an enteric coating layer containing triethyl citrate, still further coated with an enteric coating layer containing polyethylene glycol, and still yet further coated with mannitol, to give fine granules, which are mixed with additives and shaped.

The above-mentioned "enteric coating layer" includes, for example, aqueous enteric polymer substrates such as cellulose acetate phthalate (CAP), hydroxypropylmethyl cellulose phthalate, hydroxymethyl cellulose acetate succinate, methacrylic acid copolymers [e.g., Eudragit L30D-55 (trade name; produced by Rohm), Colicoat MAE30DP (trade name; produced by BASF), Polykid PA30 (trade name; produced by Sanyo Chemical)], carboxymethylethyl cellulose and shellac; sustained-release substrates such as methacrylic acid polymers [e.g., Eudragit NE30D (trade name), Eudragit RL30D (trade name), Eudragit RS30D (trade name), etc.]; water-soluble polymers; plasticizers such as triethyl citrate, polyethylene glycol, acetylated monoglycerides, triacetine and castor oil; and mixtures thereof.

The above-mentioned "additive" includes, for example, water-soluble sugar alcohols (e.g., sorbitol, mannitol, multitol, reduced starch saccharides, xylitol, reduced paratinose, erythritol, etc.), crystalline cellulose [e.g., Ceolas KG 801, Avicel PH 101, Avicel PH 102, Avicel PH 301, Avicel PH 302, Avicel RC-591 (crystalline cellulose·carmellose sodium)], low-substituted hydroxypropyl cellulose [e.g., LH-22, LH-32, LH-23, LH-33 (Shin-Etsu Chemical) and mixtures thereof]; binders, souring agents, bubbling agents, sweetening agents, flavorings, lubricants, coloring agents, stabilizers, excipients, disintegrants etc. are also used.

As a preparation using the crystal of the present invention, for example, a tablet for sustained release of the active ingredient according to WO2004-035020 or a capsule containing granules or fine granules can be employed.

The crystal of the present invention may be used in combination with 1 to 3 other active ingredients.

Such "other active ingredients" include, for example, anti-*Helicobacter* pylori activity substances, imidazole compounds, bismuth salts, quinolone compounds, and so forth. Of these substances, preferred are anti-*Helicobacter* pylori action substances, imidazole compounds etc.
Such "anti-*Helicobacter* pylori action substances" include, for example, antibiotic penicillins (e.g., amoxicillin, benzylpenicillin, piperacillin, mecillinam, etc.), antibiotic cefems (e.g., cefixime, cefaclor, etc.), antibiotic macrolides (e.g., erythromycin, clarithromycin. etc.), antibiotic tetracyclines (e.g., tetracycline, minocycline, streptomycin, etc.), antibiotic aminoglycosides (e.g., gentamicin, amikacin, etc.), imipenem, and so forth. Of these substances, preferred are antibiotic penicillins, antibiotic macrolides etc. Such "imidazole compounds" include, for example, metronidazole, miconazole, etc. Such "bismuth salts" include, for example, bismuth acetate, bismuth citrate, etc. Such "quinolone compounds" include, for example, ofloxacin, ciploxacin, etc.

Such "other active ingredients" and the crystal of the present invention may also be used in combination as a mixture prepared as a single pharmaceutical composition [e.g., tablets, powders, granules, capsules (including soft capsules), liquids, injectable preparations, suppositories, sustained-release preparations, etc.], in accordance with a commonly known method, and may also be prepared as separate preparations and administered to the same subject simultaneously or at a time interval.

### EXAMPLES

The present invention is hereinafter described in more detail by means of, but is not limited to, the following reference examples, examples and experimental examples.

In the following examples, the term "room temperature" and "ambient temperature" indicate about 15 to 30 °C.

Melting points were measured using the Micro Melting Point Apparatus (produced by Yanagimoto Seisakusho), and uncorrected values are shown.

X-ray powder diffraction was measured using Shimadzu XRD-6000 (Cu-Kα ray: λ==1.5418Å, tube voltage: 40 kV, tube current: 40 mA) or RINT Ultima⁺ 2100U (Cu-Kα ray: λ=1.5418Å, tube voltage: 40 kV, tube current: 50 mA). In case of using Shimadzu XRD-6000, the divergence and scattering slits were set at 1° and the receiving slit was set at 0.15 mm. Diffracted radiation was detected by a NaI scintillation detector. A θ-2 θ continuous scan at 3°/min (0.4 sec/0.02°step) from 2.5 to 40°2 θ was used. A silicon standard was analyzed to check the instrument alignment. Data were collected and analyzed using XRD-6100/7000 v.5.0.

FT-Raman spectrums were measured using Thermo Nicolet FT-Raman 960 spectrometer (pumped laser: 1064 nm, laser power: 0.5 to 1.5 W, spectrum range: 3500 to 100 cm⁻¹, detector: InGaAs).

Solid ¹³C-NMR spectrums (CP/MAS method) were measured using Varian Unity-INOVA 400 NMR spectrometer [¹³C nuclear resonance frequency: 100.543 MHz, sample container: 4 mm pencil-shaped zirconia rotor, measurement temperature: room temperature, MAS rotation number: 12000 Hz, standard substance: glycine solution (176.5 ppm), cumulated number: 200].

For thermogravimetric analysis, TA Instruments differential scanning calorimeter 2920 or Seiko Instruments TG/DTA 220 was used for the measurement.

Amorphous (*R*)-2-[[[3-methyl-4-(2,2,2-trifluoroethoxy)-2-pyridinyl]methyl]sulfinyl]-1H-benzimidazole to be used was prepared according to JP-A-2001-058990, Reference Example 1.

Anhydrous crystal of (*R*)-2-[[[3-methyl-4-(2,2,2-trifluoroethoxy)-2-pyridinyl]methyl]sulfinyl]-1H-benzimidazole (hereinafter referred to as Form I crystal) to be used as a starting material was prepared according to JP-A-2001-058990, Example 2.

### Example 1

### (R)-2-[[[3-methyl-4-(2,2,2-trifluoroethoxy)-2-pyridinyl]methyl]sulfinyl]-1H-bensimidazole (R(+)-lansoprazole) 1.5 hydrate crystal (Form II crystal)

Sufficient amount of Form I crystal was added to a mixture of water (1.8mL) and acetone (0.2mL) in an amber vial such that excess solid remained. The vial was capped and the mixture was agitated by constant rotation on a slurry wheel for three days at ambient temperature. Solid was then collected by filtration. As a result of thermogravimetric analysis, about 7% of weight decrease was observed at 24 to 84°C, and the crystal was assumed to be 1.5 hydrate crystal (theoretical amount of water: 6.6%).

**Table 1: XRPD data (Form II crystal)**

| **2θ (°)** | **d-value (Å)** | **Relative intensity (%)** |
|---|---|---|
| 9.1808 | 9.62491 | 41 |
| 9.9319 | 8.89865 | 42 |
| 10.9561 | 8.06898 | 34 |
| 13.3524 | 6.62577 | 28 |
| 14.74 | 6.005 | 36 |
| 14.94 | 5.92506 | 49 |
| 15.6477 | 5.65865 | 100 |
| 17.5995 | 5.03525 | 64 |
| 19.7104 | 4.5005 | 30 |
| 25.3684 | 3.50811 | 24 |
| 29.7424 | 3.0014 | 32 |

### Example 2

### (R)-2-[[[3-methyl-4-(2,2,2-trifluoroethoxy)-2-pyridinyl]methyl]sulfinyl]-1H-benzimidazole (R(+)-lansoprazole) 0.7 ethanol·1 hydrate crystal (Form III crystal)

Form I crystal was added to a mixture of water (0.05mL) and ethanol (0.45mL) in an amber vial and the solid was slowly dissolved. Addition of Form I crystals was continued until excess solid remained. At this point, a large amount of solid precipitated from solution. Solid was then collected by filtration. As a result of thermogravimetric analysis, about 7% of weight decrease was observed at 25 to 66°C. From the lattice constant calculated from the crystal structure and the results of gas chromatography analysis and the like, the crystal was assumed to be 0.7 ethanol·1 hydrate crystal (theoretical amount of ethanol: 8.1%, theoretical amount of water: 4.7%).

**Table 2: XRPD data (Form III crystal)**

| **2θ(°)** | **d-value (Å)** | **Relative intensity (%)** |
|---|---|---|
| 6.6746 | 13.23223 | 100 |
| 14.2592 | 6.20639 | 53 |
| 17.5 | 5.06365 | 17 |
| 17.82 | 4.97344 | 22 |
| 18.6484 | 4.75433 | 31 |
| 19.6535 | 4.5134 | 41 |
| 20.12 | 4.40979 | 25 |
| 20.52 | 4.32473 | 19 |

### Example 3

### (R)-2-[[[3-methyl-4-(2,2,2-trifluoroethoxy)-2-pyridinyl]methyl]sulfinyl]-1H-benzimidazole (R(+)-lansoprazole) 0.7 isopropanol· 1.2 hydrate crystal (Form IV crystal)

Form I crystal (28.8mg) was added to isopropanol (0.75mL) and the mixture was sonicated to aid dissolution. The solid was dissolved to form a clear yellow solution, which was filtered through a 0.2µm nylon filter into a clean vial. The uncovered vial was left to evaporate the filtrate under ambient condition. White needles were collected after four days. As a result of thermogravimetric analysis, about 7% of weight decrease was observed at 25 to 71°C. From the lattice constant calculated from the crystal structure and the results of gas chromatography analysis and the like, the crystal was assumed to be 0.7 isopropanol· 1.2 hydrate crystal (theoretical amount of isopropanol: 9.9%, theoretical amount of water: 5.6%).

**Table 3: XRPD data (Form IV crystal)**

| **2θ(°)** | **d-value (Å)** | **Relative intensity (%)** |
|---|---|---|
| 5.9277 | 14.89773 | 81 |
| 13.2833 | 6.66008 | 21 |
| 17.6762 | 5.01357 | 100 |
| 19.4312 | 4.56453 | 51 |
| 19.72 | 4.49833 | 20 |
| 20.3666 | 4.35695 | 34 |
| 20.85 | 4.25702 | 49 |
| 22.7916 | 3.89857 | 22 |
| 24.51 | 3.62899 | 22 |
| 25.4232 | 3.50067 | 41 |

### Example 4

### (R)-2-[[[3-methyl-4-(2,2,2-trifluoroethoxy)-2-pyridinyl]methyl]sulfinyl]-1H-benzinidazole (R(+)-lansoprazole) hydrate crystal (Pattern V crystal)

Sufficient amount of Form I crystal was added to a mixture of water (1.8mL) and ethanol (0.2mL) in an amber vial such that excess solid remained. The vial was capped and the mixture was agitated by constant rotation on a slurry wheel at ambient temperature for three days. Solid was then collected by filtration. As a result of thermogravimetric analysis, about 6% of weight decrease was observed at 17 to 62°C and as a result of thermogravimetry-infrared spectrum analysis, the presence of water was confirmed. However, a sample necessary for identification could not be obtained (theoretically-estimated amount of water: 6.8%).

**Table 4: XRPD data (Pattern V crystal)**

| **2-θ (°)** | **d-value (Å)** | **Relative intensity (%)** | **2-θ (°)** | **d-value (Å)** | **Relative intensity (%)** |
|---|---|---|---|---|---|
| 4.4079 | 20.03032 | 40 | 21.5 | 4.12976 | 41 |
| 6.6585 | 13.26418 | 20 | 21.72 | 4.08843 | 41 |
| 9.3 | 9.50181 | 21 | 22.5991 | 3.93134 | 26 |
| 9.5977 | 9.20774 | 92 | 24.12 | 3.68678 | 32 |
| 10.6447 | 8.30432 | 51 | 24.32 | 3.65691 | 48 |
| 13 | 6.80458 | 26 | 24.56 | 3.62171 | 39 |
| 13.2025 | 6.70066 | 67 | 25.66 | 3.4689 | 31 |
| 14.4698 | 6.11652 | 62 | 26.06 | 3.41655 | 43 |
| 15.0576 | 5.87905 | 87 | 26.24 | 3.39352 | 41 |
| 15.5133 | 5.70737 | 50 | 27.76 | 3.21107 | 22 |
| 15.76 | 5.61858 | 25 | 28.0247 | 3.18134 | 52 |
| 18.18 | 4.87576 | 29 | 28.36 | 3.14448 | 32 |
| 18.3622 | 4.82779 | 100 | 28.6 | 3.11864 | 23 |
| 18.84 | 4.70641 | 52 | 28.82 | 3.09533 | 24 |
| 19.02 | 4.66228 | 64 | 32.351 | 2.76509 | 21 |
| 19.716 | 4.49923 | 24 | 33.55 | 2.66896 | 32 |
| 20.1575 | 4.40167 | 72 | 36.92 | 2.43271 | 21 |
| 21.42 | 4.14501 | 43 | 37.06 | 2.42384 | 23 |

### Example 5

### (R)-2-[[[3-methyl-4-(2,2,2-trifluoroethoxy)-2-pyridinyl]methyl]sulfinyl]-1H-benzimidazole (R(+)-lansoprazole) 0.5 hydrate crystal (Form VI crystal)

Form II crystal (Example 1) was placed in a vacuum oven and dried overnight at ambient temperature under oil-pump vacuum. The solid was then removed from the oven. As a result of thermogravimetric analysis, about 3% of weight decrease was observed at 25 to 55°C, and the crystal was assumed to be 0.5 hydrate crystal (theoretical amount of water: 3.1%).

**Table 5: XRPD data (Form VI crystal)**

| **2-θ(°)** | **d-value (Å)** | **Relative intensity (%)** |
|---|---|---|
| 9.9762 | 8.85923 | 60 |
| 10.4902 | 8.42627 | 30 |
| 15.81 | 5.60092 | 32 |
| 16.9644 | 5.2223 | 100 |
| 18.3461 | 4.83199 | 28 |
| 21.0793 | 4.21122 | 25 |

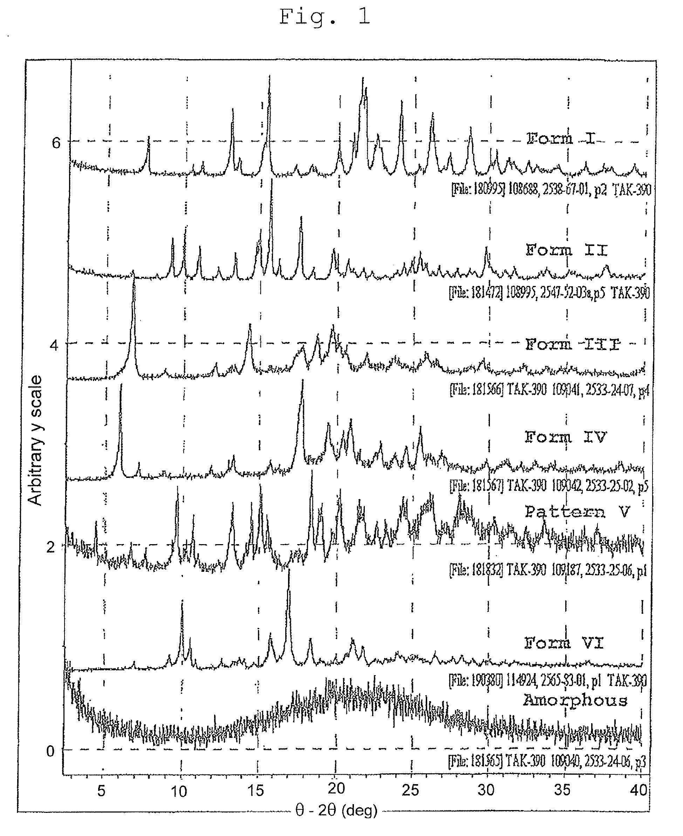

The X-ray powder diffraction patterns of Form II crystal, Form III crystal, Form IV crystal, Pattern V crystal and Form VI crystal are shown in Figure 1 along with the patterns of Form I crystal and amorphous form thereof.

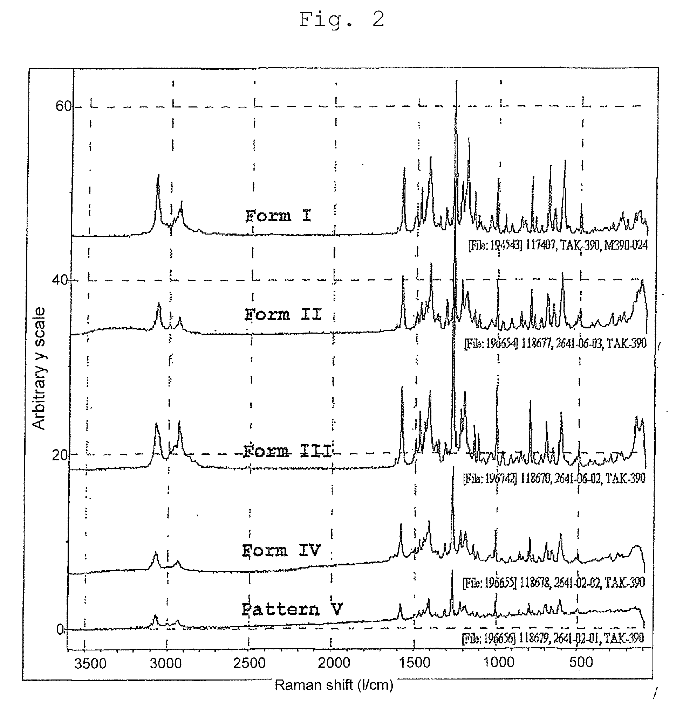

The FT-Raman spectrums of Form II crystal, Form III crystal, Form IV crystal and Pattern V crystal are shown in Figure 2 along with the spectrum of Form I crystal.

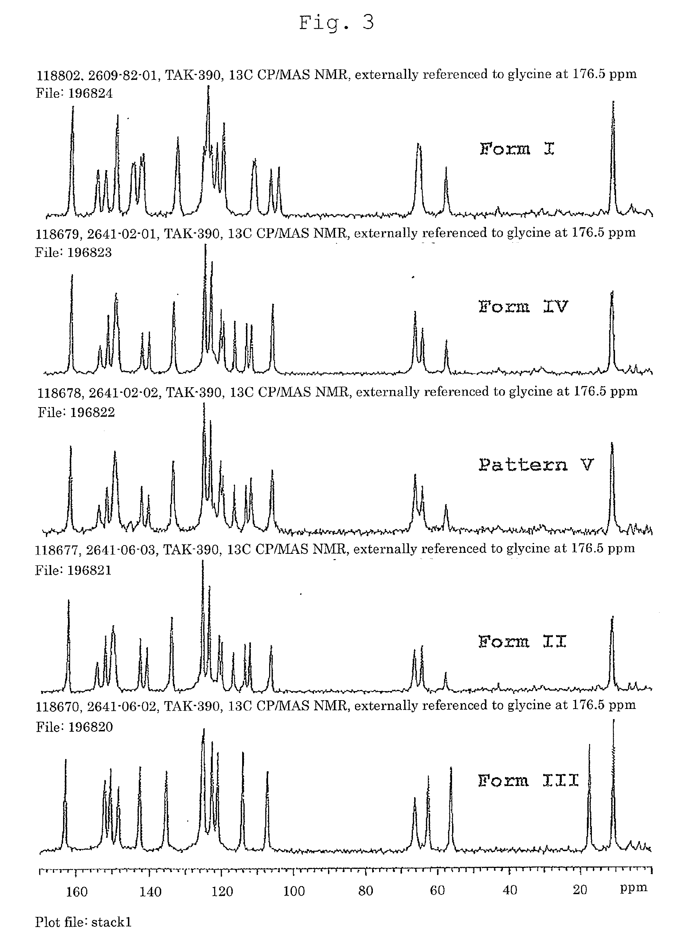

The solid ¹³C-NMR spectrums of Form II crystal, Form III crystal, Form IV crystal and Pattern V crystal are shown in Figure 3 along with the spectrum of Form I crystal.

### Example 6

### (R)-2-[[[3-methyl-4-(2,2,2-trifluoroethoxy)-2-pyridinyl]methyl]sulfinyl]-1H-benzimidazole (R(+)-lansoprazole) methanol solvate crystal

Form I crystal (100ml) was placed in a test tube, methanol was added at room temperature and the crystal was dissolved in an essentially minimum amount and diluted about 3-fold. The solution (2mL) was spread thin in a weighing bottle (diameter about 30mm), and left standing without capping at -20°C to allow gradual crystallization. Thereafter, the solid was collected by filtration. As a result of thermogravimetric analysis, weight decrease was observed from immediately after temperature rise. However, since the decreased weight was not clear, the crystal was assumed to be methanol solvate but not a clear solvate containing methanol in a given mol number.

**Table 6: XRPD data (methanol solvate crystal)**

| **2θ(°)** | **d-value (Å)** | **Relative intensity (%)** | **2θ (°)** | **d-value (Å)** | **Relative intensity (%)** |
|---|---|---|---|---|---|
| 6.200 | 14.2437 | 12 | 18.700 | 4.7412 | 29 |
| 6.280 | 14.0624 | 20 | 19.580 | 4.5301 | 21 |
| 6.420 | 13.7561 | 29 | 19.680 | 4.5073 | 35 |
| 6.580 | 13.4219 | 80 | 20.140 | 4.4054 | 22 |
| 6.680 | 13.2212 | 100 | 20.560 | 4.3163 | 42 |
| 8.720 | 10.1322 | 13 | 21.500 | 4.1297 | 15 |
| 12.080 | 7.3205 | 15 | 21.660 | 4.0995 | 21 |
| 14.180 | 6.2407 | 20 | 21.760 | 4.0809 | 18 |
| 14.240 | 6.2146 | 35 | 22.240 | 3.9939 | 19 |
| 14.360 | 6.1629 | 50 | 22.340 | 3.9762 | 23 |
| 15.720 | 5.6326 | 13 | 23.860 | 3.7263 | 18 |
| 17.280 | 5.1275 | 16 | 24.440 | 3.6391 | 19 |
| 17.520 | 5.0578 | 19 | 25.920 | 3.4346 | 15 |
| 17.820 | 4.9733 | 23 | 26.120 | 3.4088 | 19 |
| 18.140 | 4.8863 | 19 | 26.560 | 3.3533 | 22 |
| 18.240 | 4.8697 | 33 | 26.600 | 3.3483 | 17 |

### Example 7

### (R)-2-[[[3-methyl-4-(2,2,2-trifluoroethoxy)-2-pyridinyl]methyl]sulfinyl]-1H-benzimidazole (R(+)-lansoprazole) ethanol solvate crystal

Form I crystal (100ml) was placed in a test tube, methanol was added at room temperature and the crystal was dissolved in an essentially minimum amount and diluted about 3-fold. The solution (2mL) was spread thin in a weighing bottle (diameter about 30mm), and left standing without capping at -20°C to allow gradual crystallization. Thereafter, the solid was collected by filtration. As a result of thermogravimetric analysis, weight decrease was observed from immediately after temperature rise. However, since the decreased weight was not clear, the crystal was assumed to be ethanol solvate but not a clear solvate containing ethanol in a given mol number.

**Table 7: XRPD data (ethanol solvate crystal)**

| **2θ(°)** | **d-value (Å)** | **Relative intensity (%)** | **2θ(°)** | **d-value** | **Relative intensity (Å) (%)** |
|---|---|---|---|---|---|
| 6.180 | 14.2897 | 17 | 19.680 | 4.5073 | 24 |
| 6.360 | 13.8857 | 26 | 19.740 | 4.4937 | 25 |
| 6.440 | 13.7134 | 36 | 19.960 | 4.4447 | 15 |
| 6.540 | 13.5039 | 58 | 20.220 | 4.3881 | 19 |
| 6.680 | 13.2212 | 100 | 20.380 | 4,3540 | 16 |
| 6.760 | 13.0649 | 40 | 20.480 | 4,3330 | 25 |
| 8.760 | 10.0860 | 13 | 20.540 | 4.3205 | 30 |
| 12.080 | 7.3205 | 15 | 20.600 | 4.3080 | 30 |
| 14.220 | 6.2233 | 31 | 21.720 | 4.0883 | 19 |
| 14.360 | 6.1629 | 37 | 21.800 | 4.0735 | 20 |
| 17.240 | 5.1393 | 15 | 22.380 | 3.9692 | 17 |
| 17.400 | 5.0924 | 19 | 22.480 | 3.9518 | 14 |
| 17.780 | 4.9844 | 24 | 23.680 | 3.7542 | 22 |
| 17.840 | 4.9678 | 21 | 23.740 | 3.7448 | 21 |
| 18.180 | 4.8756 | 16 | 24.500 | 3.6304 | 14 |
| 18.300 | 4.8439 | 24 | 25.860 | 3.4424 | 14 |
| 18.540 | 4.7818 | 23 | 25.940 | 3.4320 | 14 |
| 18.720 | 4.7362 | 30 | 26.020 | 3.4216 | 14 |
| 19.080 | 4.6476 | 16 | 26.320 | 3.3833 | 14 |
| 19.200 | 4.6189 | 22 | 26.520 | 3.3582 | 21 |
| 19.360 | 4.5810 | 17 | 26.580 | 3.3508 | 19 |
| 19.580 | 4.5301 | 19 | 26.620 | 3.3459 | 15 |
| 19.640 | 4.5164 | 25 | 26.700 | 3.3360 | 13 |
| | | | 29.460 | 3.0294 | 13 |

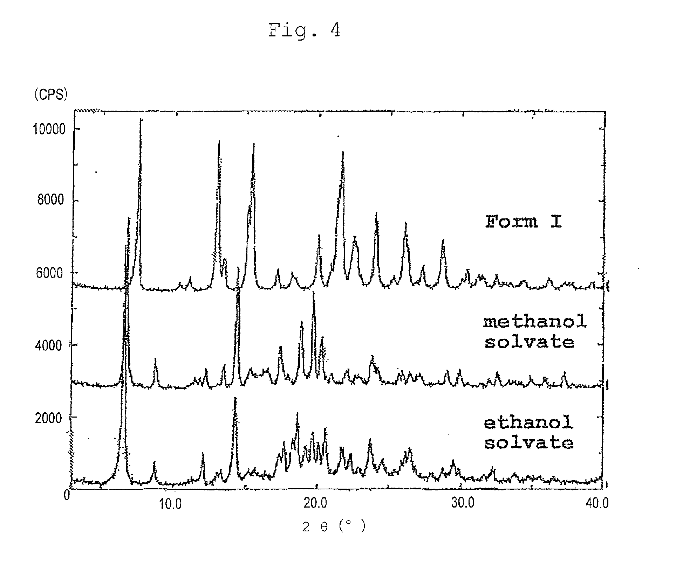

The X-ray powder diffraction patterns of methanol solvate crystal and ethanol solvate crystal are shown in Figure 4 along with the pattern of Form I crystal.

### Example 8

### (R)-2-[[[3-methyl-4-(2,2,2-trifluoroethoxy)-2-pyridinyl]methyl]sulfinyl]-1H-benzimidazole (R(+)-lansoprazole) 1.0 hydrate crystal

Form I crystal (100ml) was placed in a test tube, a mixed solvent of water and methanol (1:1) was added at room temperature and the crystal was dissolved in an essentially minimum amount. The solution was left standing as it was at - 20°C to allow crystallization. Thereafter, the solid was collected by filtration. As a result of thermogravimetric analysis, about 6.5% of weight decrease was observed at about 40°C to 80°C, and the crystal was assumed to be 1.5 hydrate. This was dried under reduced pressure for 3 days using a rotary pump. The resulting sample was subjected to thermogravimetric analysis. As a result, the weight decrease at about 40°C to 80°C decreased to 4.4%, and the crystal was assumed to have changed from 1.5 hydrate to 1.0 hydrate.

**Table 8: XRPD data (1.0 hydrate crystal)**

| **2θ (°)** | **d-value (Å)** | **Relative intensity** | **2θ(°)** | **d-value (Å)** | **Relative intensity (%)** |
|---|---|---|---|---|---|
| 9.040 | 9.7743 | 17 | 21.460 | 4.1373 | 18 |
| 9.100 | 9.7100 | 18 | 21.620 | 4.1070 | 34 |
| 9.900 | 8.9270 | 46 | 21.680 | 4.0958 | 36 |
| 10.440 | 8.4665 | 47 | 21.760 | 4.0809 | 27 |
| 15.680 | 5.6469 | 45 | 21.820 | 4.0698 | 19 |
| 15.740 | 5.6255 | 41 | 22.340 | 3.9762 | 17 |
| 15.800 | 5.6043 | 33 | 22.480 | 3.9518 | 19 |
| 16.800 | 5.2481 | 100 | 24.180 | 3.6777 | 21 |
| 18.240 | 4.8597 | 31 | 24.400 | 3.5450 | 17 |
| 18.280 | 4.8492 | 31 | 25.180 | 3.5338 | 18 |
| 18.360 | 4.8282 | 28 | 26.320 | 3.3833 | 20 |
| 18.420 | 4.8126 | 18 | 26.480 | 3.3632 | 25 |
| 19.920 | 4.4535 | 16 | 27.580 | 3.2315 | 17 |
| 20.600 | 4.3080 | 18 | 28.240 | 3.1575 | 18 |
| 20.900 | 4.2468 | 29 | 28.900 | 3.0869 | 17 |
| 20.980 | 4.2308 | 36 | 28.940 | 3.0827 | 16 |
| 21.380 | 4.1526 | 17 | | | |

### Example 9

### (R)-2-[[[3-methyl-4-(2,2,2-trifluoroethoxy)-2-pyridinyl]methyl]sulfinyl]-1H-benzimidazole (R(+)-lansoprazole) 1.5 hydrate crystal

Form I crystal (100ml) was placed in a test tube, a mixed solvent of water and methanol (1:1) was added at room temperature and the crystal was dissolved in an essentially minimum amount. The solution was left standing as it was at - 20°C to allow crystallization. Thereafter, the solid was collected by filtration. As a result of thermogravimetric analysis, about 6.5% of weight decrease was observed at about 40°C to 80°C, and the crystal was assumed to be 1.5 hydrate.

**Table 9: XRPD data (1.5 hydrate crystal)**

| **2θ(°)** | **d-value (Å)** | **Relative (%)** | **2θ(°)** | **d-value (Å)** | **Relative intensity (%)** |
|---|---|---|---|---|---|
| 6.700 | 13.1818 | 13 | 21.480 | 4.1335 | 17 |
| 9.200 | 9.6046 | 38 | 21.580 | 4.1145 | 28 |
| 9.740 | 9.0733 | 14 | 22.260 | 3.9904 | 26 |
| 9.800 | 9.0179 | 23 | 22.320 | 3.9798 | 20 |
| 9.960 | 8.8734 | 40 | 23.720 | 3.7479 | 17 |
| 11.000 | 8.0367 | 46 | 23.820 | 3.7324 | 20 |
| 13.420 | 6.5924 | 28 | 23.900 | 3,7201 | 25 |
| 14.760 | 5.9968 | 41 | 23.960 | 3.7109 | 27 |
| 14.820 | 5.9726 | 43 | 24.300 | 3.6598 | 25 |
| 14.880 | 5.9487 | 47 | 24.380 | 3.6480 | 27 |
| 14.980 | 5.9092 | 60 | 24.460 | 3.6362 | 19 |
| 15.480 | 5.7194 | 28 | 24.940 | 3.5673 | 25 |
| 15.680 | 5.6469 | 100 | 25.340 | 3.5119 | 18 |
| 16.200 | 5.4668 | 17 | 25.380 | 3.5064 | 21 |
| 16.320 | 5.4269 | 15 | 25.480 | 3.4929 | 18 |
| 17.540 | 5.0521 | 32 | 25.720 | 3.4609 | 17 |
| 17.640 | 5.0237 | 44 | 26.180 | 3.4011 | 17 |
| 17.720 | 5.0012 | 29 | 27.140 | 3.2829 | 22 |
| 19.660 | 4.5118 | 58 | 27.200 | 3.2758 | 24 |
| 19.700 | 4.5027 | 63 | 28.260 | 3.1553 | 16 |
| 19.840 | 4.4713 | 28 | 28.960 | 3.0806 | 18 |
| 19.900 | 4.4579 | 16 | 29.740 | 3.0016 | 18 |
| 20.840 | 4.2589 | 19 | 29.840 | 2.9917 | 17 |
| 21.240 | 4.1796 | 18 | 31.080 | 2.8751 | 17 |

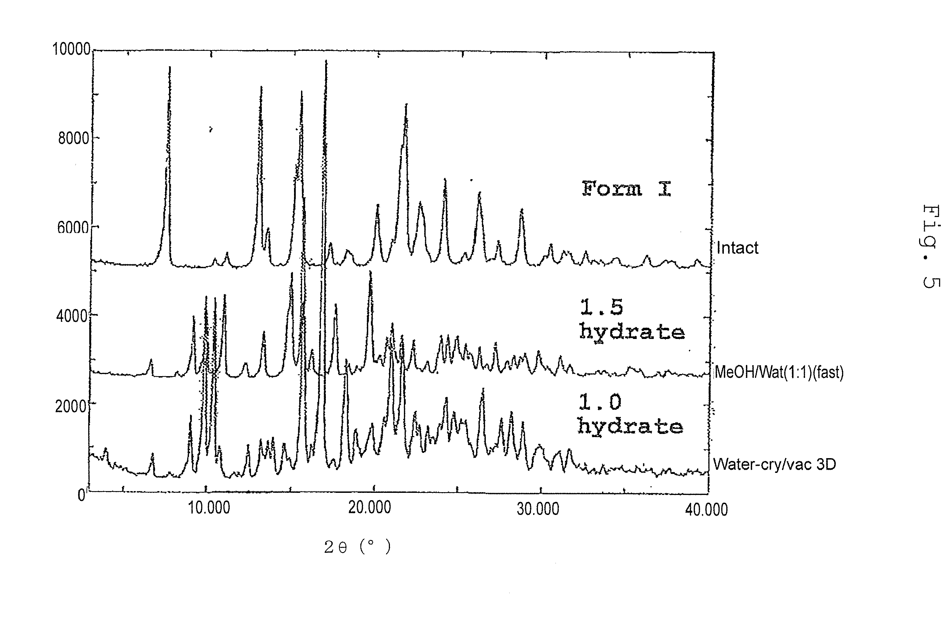

The X-ray powder diffraction patterns of 1.0 hydrate crystal and 1.5 hydrate crystal are shown in Figure 5 along with the pattern of Form I crystal.

### Experimental Example 1: Solubility

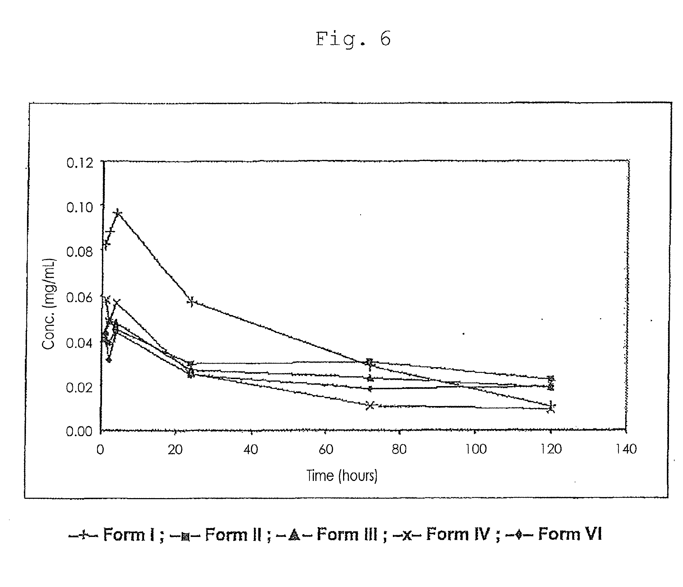

The solubilities of Forms I, II, III, IV and VI of (R)-lansoprazole obtained in the above-mentioned Reference Example 1 and Examples were tested as a suspension of power in water at 25°C for 5 days. The solid samples of each form were tested as-is by HPLC and XRPD without particle size determination. Forms II, III, IV and VI exhibited similar solubility, and were chemically degraded over time (see the solubility chart below Figure 6). The extent of chemical degradation as a function of time was similar for all forms. After the solubility study, the residual solids were analyzed by XRPD, and showed that all convereted to Form II except for the Form I sample. The Form I sample from the solubility study analyzed by XRPD was a mixture consisting of Form I (major component) and Form II.

### Experimental Example 2: Relationships among Forms of R(+)-lansoprazole

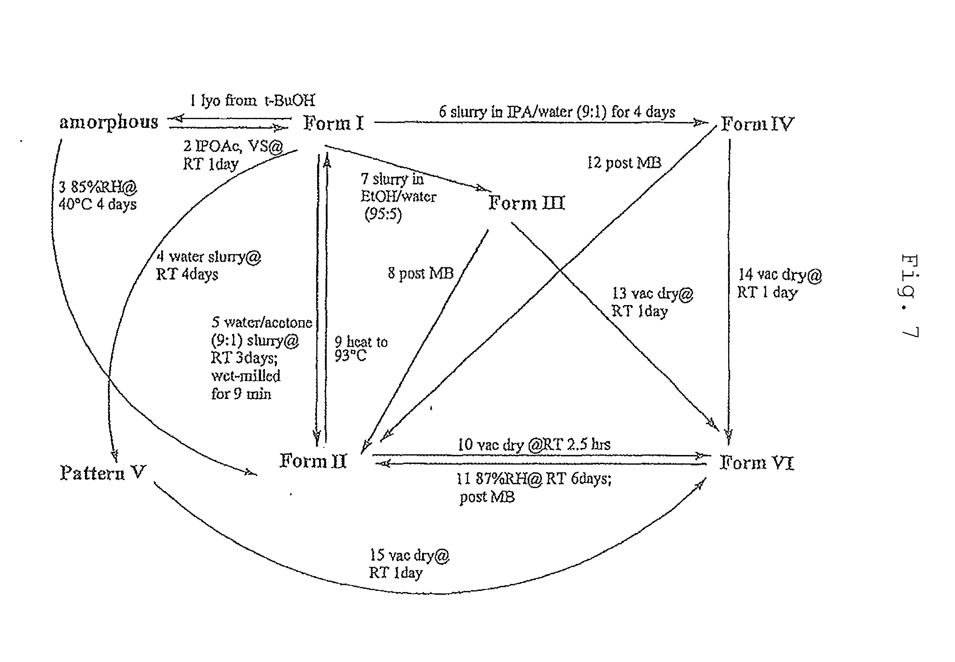

The relationships among Forms of *R*(+)-lansoprazole were studied under various conditions. The results are shown in Figure 7. The conditions are shown in Table 10.

**Table 10**

| Reaction | Conditions^{a} |
|---|---|
| 1 | Form I (180.6 mg) was dissolved upon sonication in 18 mL of t-BuOH and filtered through a 0.2 mm nylon filter into a flask. The solution was frozen in a dry ice/acetone bath and lyophilized for 1 day. |
| 2 | Amorphous (*R*)-lansoprazole (from lyophilization) was placed into a vial. The uncapped vial was then placed inside a larger amber vial containing 1 mL IPOAc. The larger vial was capped and left for one day at RT. |
| 3 | Amorphous (*R*)-lansoprazole (from lyophilization) was placed into a vial. The uncapped vial was placed into an 85%RH chamber. The chamber was placed in a 40°C oven for 4 days. |
| 4 | 20 mL of water was added to Form I (19.7 mg) and the mixture was sonicated. Solids remained after sonication. The vial was capped and wrapped in aluminum foil, and placed on rotating wheel and slurried at RT for 4 days. |
| 5 | 1.8 mL (2×0.9 mL) of water and 0.2 mL of acetone (water/acetone (9:1)) were added to Form I. Solids remained and slurried on a rotating wheel at RT for 3 days. |
| | Form I was placed into a ceramic milling jar. 10 µL of water and a ceramic ball were added, and the jar was capped. The sample was milled for a total of nine minutes (3×3 min cycle). Solids were scraped and allowed to cool between cycles. Solids were collected in a vial and refrigerated. |
| 6 | 8 ml (2x4 ml) of IPA/water (9:1) was added to Form I (2.54489 g). Solids remained, capped with PTFE cap and slurried on a rotating wheel for 4 days. Solids were vacuum filtered and spread onto a etri dish, covered with kimwipe paper, and allowed to dry for 1 day. Solids were collected in a vial after 1 day and capped. |
| 7 | 5 ml of EtOH/water (95:5) was added to Form I (2.45288 g). Solids remained, capped with PTFE cap and slurried on a rotating wheel for 1 day. Sample appeared as a deep red/purple paste. Sample was spread onto a etri dish, covered and allowed to dry in a hood. |
| 8 | Form III post DVS. Moisture sorption/desorption data were collected on a VTI SGA-100 Vapor Sorption Analyzer. Sorption and desorption data were collected over a range of 5% to 95% relative humidity (RH) at 10% RH intervals under a nitrogen purge. Samples were not dried prior to analysis. Equilibrium criteria used for analysis were less than 0.0100% weight change in 5 minutes, with a maximum equilibration time of 3 hours if the weight criterion was not met. Data were not corrected for the initial moisture content of the samples. NaCl and PVP were used as calibration standards. Starting amount of Form III was 11.9 mg. |
| 9 | Form II was placed into a vial. The vial was purged with nitrogen and heated to 93°C in an oil bath. Sample had turned brown in up to 45 seconds, removed from oil bath after up to 1 min. |
| 10 | Form II was vacuum dried in an open vial at RT for up to 2.5 hours. |
| 11 | Form VI was placed into a vial. The uncapped vial was placed into an 87% RH chamber at RT for 6 days. |
| | Form VI post DVS. Moisture sorption/desorption data were collected on a VTI SGA-100 Vapor Sorption Analyzer. Sorption and desorption data were collected over a range of 5% to 95% relative humidity (RH) at 10% RH intervals under a nitrogen purge. Samples were not dried prior to analysis. Equilibrium criteria used for analysis were less than 0.0100% weight change in 5 minutes, with a maximum equilibration time of 3 hours if the weight criterion was not met. Data were not corrected for the initial moisture content of the samples. NaCl and PVP were used as calibration standards. Starting amount of Form VI was 6.6 mg. |
| 12 | Form IV post DVS. Moisture sorption/desorption data were collected on a VTI SGA-100 Vapor Sorption Analyzer. Sorption and desorption data were collected over a range of 5% to 95% relative humidity (RH) at 10% RH intervals under a nitrogen purge. Samples were not dried prior to analysis. Equilibrium criteria used for analysis were less than 0.0100% weight change in 5 minutes, with a maximum equilibration time of 3 hours if the weight criterion was not met. Data were not corrected for the initial moisture content of the samples. NaCl and PVP were used as calibration standards. Starting amount of Form IV was 9.4 mg. |
| 13 | Form III was placed into a vial. The uncapped vial was placed into a vacuum oven (at RT) for 1 day. |
| 14 | Form IV was placed into a vial. The uncapped vial was placed into a vacuum oven (at RT) for 1 day. |
| 15 | Pattern V was placed into a vial. The uncapped vial was placed into a vacuum oven (at RT) for 1 day. |

| | |
|---|---|
| ^{a} EtOH = ethanol, IPA = isopropanol, IPOAc = isopropyl acetate, DVS = dynamic vapor sorption, RH = relative humidity, RT = room temperature, t-BuOH = tert-butanol, NaCl = sodium chloride, PVP = polyvinylpyrrolidone. | |

### Experimental Example 3 Interconversion Slurries

Mixtures of Forms I, II, III, IV and VI were slurried in aqueous saturated solutions at ambient temperature and up to 40°C. Table 11 summarizes the results.

A mixture of Form II and Pattern V material was obtained when the mixture of forms was slurried at ambient temperature for 5 days, despite the absence of Pattern V material as starting material. However, additional slurry time under the same conditions produced Form II exclusively, suggesting that Pattern V material converted to Form II. However, data presented in Table 12 associated with the preparation of Forms suggests that Pattern V material can be present as a mixture with Form II over a long period of time.

Form II was obtained exclusively when the mixture of Forms I, II, III, IV and VI was slurried at up to 40°C for 5 days.

**Table 11: Interconversion Slurries**

| Starting Forms present | Conditions | Slurry time | XRPD Result |
|---|---|---|---|
| I, II, III, IV, VI | Slurry in water, ambient | 5 days | II+V |
| I, II, III, IV, VI | Slurry in water, ambient | 9 days | II |
| I, II, III, IV, VI | Slurry in water, ambient | 13 days | II |
| I, II, III, IV, VI | Slurry in water, up to 40°C | 5 days | II |

**Table 12: Preparation of Large Scale Samples**

| Intended Form | Conditions | XRPD Result |
|---|---|---|
| II | Slurry in water:acetone 9:1, 4 days | II |
| III | Slurry in ethanol:water 95:5, 1 day | III |
| | Spontaneous precipitation from ethanol:water 95:5 | |
| IV | Slurry in isopropanol:water 9:1, 4 days | IV |
| | Slurry in isopropanol:water 9:1, 1 day | |
| V | Slurry in water:ethanol 9:1, 4 days | II |
| | Slurry of Form II in water:ethanol 9:1, 5 days | II |
| | Slurry of Form II in water: ethanol 9:1, up to 1 month | II |
| | Slurry of Form II in water:ethanol 9:1, up to 1 month | |
| | Slurry in water, 1 day | II |
| | Slurry in water, 5 days | I + II |
| | Slurry in water, up to 1 month | II + V |
| | Slurry in water, 51 days | - |
| VI | Drying of Form II under vaccum at ambient, 1 day | VI |

According to Experimental Example 2 and 3, it is suggested that stable crystals in transfer are Form II, Form VI, Form III, Form IV, and Form V, in that order.

Since the crystal of the present invention has excellent antiulcer action, gastric acid secretion-inhibiting action, mucosa-protecting action, anti-Helicobacter pylori action, etc., and shows low toxicity, it is useful as a pharmaceutical product. Moreover, the crystal of the present invention shows different physical properties, particularly solubility, from those of conventional (R)-lansoprazole crystal. Solubility can markedly influence the bioavailability of pharmaceutical products. Hence, using the crystal of the present invention, a preparation design different from that of conventional crystal in solubility and the like is available, and the crystal is useful, for example, for the invention of controlled release dosage form and the like.

This application is based on provisional application No. 61/018,021 filed in the United States, the contents of which are hereby incorporated by reference.

Although the present invention have been presented or described by referring to preferred embodiments of this invention, it will, however, be understood by those of ordinary skill in the art that various modifications may be made to the forms and details without departing from the scope of the invention as set forth in the appended claims. All patents, patent publications and other publications indicated or cited in the Specification are hereby incorporated in their entireties by reference.

## Claims

1. A solvate or solvate·hydrate crystal of (R)-2-[[[3-methyl-4-(2,2,2-trifluoroethoxy)-2-pyridinyl]methyl]sulfinyl]-1H-benzimidazole, selected from:
(i) an ethanol·hydrate crystal of (R)-2-[[[3-methyl-4-(2,2,2-trifluoroethoxy)-2-pyridinyl]methyl]sulfinyl]-1H-benzimidazole wherein the X-ray powder diffraction analysis pattern has characteristic peaks at interplanar spacings (d) of 13.23±0.2, 6.21±0.2, 4.75±0.2, 4.51±0.2 and 4.41±0.2 Angstrom;
(ii) an isopropanol·hydrate crystal of (R)-2-[[[3-methyl-4-(2,2,2-trifluoroethoxy)-2-pyridinyl]methyl]sulfinyl]-1H-benzimidazole wherein the X-ray powder diffraction analysis pattern has characteristic peaks at interplanar spacings (d) of 14.90±0.2, 5.01±0.2, 4.56±0.2, 4.26±0.2 and 3.50±0.2 Angstrom;
(iii) a methanol solvate crystal of (*R*)-2-[[[3-methyl-4-(2,2,2-trifluoroethoxy)-2-pyridinyl]methyl]sulfinyl]-1H-benzimidazole wherein the X-ray powder diffraction analysis pattern has characteristic peaks at interplanar spacings (d) of 13.42±0.2, 13.22±0.2, 6.21±0.2, 6.16±0.2, 4.51±0.2 and 4.32±0.2 Angstrom; or
(iv) an ethanol solvate crystal of (*R*)-2-[[[3-methyl-4-(2,2,2-trifluoroethoxy)-2-pyridinyl]methyl]sulfinyl]-1H-benzimidazole wherein the X-ray powder diffraction analysis pattern has characteristic peaks at interplanar spacings (d) of 13.71±0.2, 13.50±0.2, 13.22±0.2, 13.06±0.2 and 6.16±0.2 Angstrom.

2. A pharmaceutical agent which comprises the crystal of claim 1.

3. A pharmaceutical agent according to claim 2, which is an agent for the prophylaxis or treatment of digestive ulcer.
